# EUROPEAN PATENT SPECIFICATION

(11) **EP 1 901 071 B1**
(45) Date of publication and mention of the grant of the patent: **10.03.2010**
(21) Application number: 06756917.8
(22) Date of filing: 02.06.2006
(51) Int. Cl.: G01N 33/576, A61K 39/395, A61P 1/16

(54) **METHODS FOR SCREENING AN ANTI-HEPATITIS C VIRUS CANDIDATE**
VERFAHREN ZUR SICHTUNG VON ANTI-HEPATITS C VIRUS KANDIDATEN
PROCÉDÉS DE CRIBLAGE DE MOLÉCULES ANTI-VIRUS DE L'HÉPATITE C

(30) Priority: 02.06.2005 JP 2005162557
(43) Date of publication of application: 19.03.2008
(73) Proprietor: Matsumori, Akira, Minoh-shi, Osaka 562-0045 (JP)
(72) Inventor: Matsumori, Akira, Minoh-shi, Osaka 562-0045 (JP)
(74) Representative: Müller-Boré & Partner Patentanwälte
(86) International application number: PCT/JP2006/311082
(87) International publication number: WO 2006/129786

(56) References cited:
- WO-A-00/26418
- WO-A-02/062956
- WO-A-02/100900
- WO-A-2004/013318
- SANSONNO D ET AL: "Detection of Hepatitis C Viurs(HCV) proteins by immunofluorescence and HCV RNA genomic sequences by non-isotopic in situ hybridization in bone marrow and peripheral blood mononuclear cells of chronically HCV-infected patients" CLINICAL AND EXPERIMENTAL IMMUNOLOGY, OXFORD, GB, vol. 103, 1 January 1996 (1996-01-01), pages 414-421, XP003002676 ISSN: 0009-9104
- ESPOSITO G ET AL: "RECOMBINANT HUMAN ANTIBODIES SPECIFIC FOR HEPATITIS C VIRUS PROTEINS" ARCHIVES OF VIROLOGY, NEW YORK, NY, US, vol. 142, no. 3, 1 January 1997 (1997-01-01), pages 601-610, XP001148378 ISSN: 0304-8608
- HEINTGES TOBIAS ET AL: "Characterization and binding of intracellular antibody fragments to the hepatitis C virus core protein" BIOCHEMICAL AND BIOPHYSICAL RESEARCH COMMUNICATIONS, vol. 263, no. 2, 24 September 1999 (1999-09-24), pages 410-418, XP002492589 ISSN: 0006-291X
- SANSONNO D. ET AL.: 'Detection and Distribution of Hepatitis C Virus-Related Proteins in Lymph Nodes of Patients With Type II Mixed Cryoglobulinemia and Neoplastic or Non-Neoplastic Lymphoproliferation' BLOOD vol. 88, no. 12, 1996, pages 4638 - 4645, XP003002675
- SANSONNO D. ET AL.: 'Detection of Hepatitis C Viurs(HCV) proteins by immunofluorescence and HCV RNA genomic sequences by non-isotopic in situ hybridization in bone marrow and peripheral blood mononuclear cells of chronically HCV-infected patients' CLIN. EXP. IMMUNOL. vol. 103, 1996, pages 414 - 421, XP003002676

## Description

### TECHNICAL FIELD

The present invention relates to a method for screening an anti-HCV drug candidate.

### BACKGROUND ART

Hepatitis C accounts for 90 to 95 % of the hepatitides attributable to blood transfusion; however, a therapeutic treatment for hepatitis C has not yet been established. For this reason, hepatitis C is one of the diseases for which urgent development of a diagnostic method, a preventive vaccine, and a therapeutic drug is in enormous demand.

The heretofore known diagnosis of hepatitis C relies on values such as GOT, GPT, LDH, etc., obtained by a blood test. However, such a diagnostic test is not sufficiently accurate or sensitive, and hence fails to enable early detection of hepatitis C.

Alternatively, HCV RNA measurement and an immunological method which uses an anti-HCV antibody are known as methods for examining the presence or absence of HCV (through virus markers such as virus proteins, virus RNAs, etc.) in the body of an infected patient. However, even these methods do not provide fully satisfying accuracy. Particularly, conventional methods, such as an immunohistological method and HCV RNA detection in tissues by RNA hybridization, pose problems in the sensitivity and specificity to the detection of HCV antigens. For this reason, these methods can only be used in extremely limited areas of application.

Using these conventional techniques, the following has been reported:
(1) a method for detecting HCV using virus RNAs (see Non-patent Documents 1-8),
(2) observation of positive hepatocytes using antibodies to HCV proteins (see Non-patent Documents 9-14),
(3) observation of positive results in, other than hepatocytes, myocardial muscle cells, epithelium cells of the kidney, glandular cells of the urinary tubule and the pancreas, etc., using antibodies to HCV proteins (see Non-patent Document 15), and
(4) detection of HCV proteins in peripheral blood, bone marrow, as well as lymphocytes, monocytes, and macrophages of lymph nodes (see Non-patent Documents 16 and 17).

However, it is not known that HCV infection in vivo develops when HCV enters leukocytes, particularly monocytes; HCV infection in vivo is preventable by arresting HCV infection in leukocytes; and HCV infection can be treated and ameliorated by removing HCV-infected leukocytes.

Partial base sequences of HCV proteins and HCV genes are already known (see Patent Document 1, and Non-patent Documents 18 and 19), and antibodies to HCV proteins are also already known. The publicly known anti-HCV antibodies include, for example, Core A-2 antibody to the HCV core protein (see Non-patent Document 20), NS4a antibody to the HCV NS4a protein (see Non-patent Document 21), etc.
[Patent Document 1] Unexamined European Patent Publication No. 318216 specification
[Non-patent Document 1] Nishiguchi, S. et al., Hepatogastroenterology, 50; 1301-1304, 2003
[Non-patent Document 2] Azzari, C. et al., Blood, 96; 2045-2048, 2000
[Non-patent Document 3] Crovatto, M. et al., Haematologica, 85; 356-361, 2000
[Non-patent Document 4] Criber, B. et al., Arch. Virol., 144; 355-364, 1999
[Non-patent Document 5] Martin, J. et al., J. Med. Virol., 54; 265-270, 1998
[Non-patent Document 6] Moonka, D. K. et al., J. Viral. Hepat., 5; 27-33, 1998
[Non-patent Document 7] Navas, S. et al., J. Virol., 1640-1646, 1998
[Non-patent Document 8] Zehender, G. et al., J. Infect. Dis., 176; 1209-1214, 1997
[Non-patent Document 9] Nepomnyaschhikh, G.I. et al., Bull. Exp. Biol. Med., 134: 307-311, 2002
[Non-patent Document 10] Nayak, N.C. et al., Acta Histochem., 101: 409-419, 1999
[Non-patent Document 11] Chamlian, A. et al. Cell Mol. Biol., 42; 557-166, 1996
[Non-patent Document 12] Zhao, X. et al., Chin. Med. J., 109; 486-488, 1996
[Non-patent Document 13] Blight, K. et al., Am. J. Pathol., 143; 1568-1573, 1993
[Non-patent Document 14] Hiramatsu, N. et al., Hepatology, 16; 306-311, 1992
[Non-patent Document 15] Yan et al., World J. Gastroenterol., 6; 805-811, 2000
[Non-patent Document 16] Sansonno, D. et al., Clin. Exp. Immunol., 103; 414-421, 1996
[Non-patent Document 17] Sansonno, D., Blood, 88; 4638-4645, 1996
[Non-patent Document 18] Okamoto et al., The Japan Journal of Experimental Medicine, Vol. 60, pages 167-177 (1990)
[Non-patent Document 19] Kato et al., Proceedings of the National Academy of Sciences of the U.S.A., Vol. 87, pages 9524-9528 (1990)
[Non-patent Document 20] Takahashi, K. et al., J. Gen. Virol., 73: 667-672, 1992
[Non-patent Document 21] Hijikata et al., Proc. Natl. Acad. Sci., USA, 90; 10773-10777, 1993

The paten WO 2004/013318 discloses a screening method for discovery, validation and further development of drugs for all stages of Hepatitis-C virus (HCV)-replication using antibodies against different proteins of HCV for visualizing level of infection a culturing system with HCV-infected PBLs or PBMCs and cells which are infectable with HCV, like e.g. naïve PBLs (monocyte-depleted PBMCs). The document suggests that said method can be used to infect cells such as monocytes but does not hint at the use of monocytes in the screening method but rather suggest the use of monocyte-depleted PBMCs (PBLs).

### DISCLOSURE OF THE INVENTION

### PROBLEMS TO BE SOLVED BY THE INVENTION

The object of the present invention is to provide a method for screening a candidate substance for an anti-HCV drug (an anti-HCV drug candidate) effective in the prevention or treatment of HCV infection.

### MEANS FOR SOLVING THE PROBLEMS

A present inventor conducted extensive studies to solve the above problems, and found that HCV proteins are localized in leukocytes, particularly in monocytes, of HCV-infected patients, and such localization often reflects HCV infection (Examples 1 and 2). The inventor studied further, and found that HCV infection in vivo develops when HCV infects leukocytes, in particular, monocytes (Example 3). In other words, these findings mean that HCV infection to the living body can be blocked (prevented) by arresting HCV infection in leukocytes. With this in mind, the present inventor examined the anti-HCV action of interferon, a conventionally known anti-HCV drug. As a result, the inventor confirmed that inteferon has an inhibitory action on HCV infection in leukocytes (Example 5 (2)) and a reducing action on the amount of HCV antigens in leukocytes (Example 4), whereby the above presumption was verified. Further, these findings also suggest that HCV infection can be treated or ameliorated by removing HCV-infected leukocytes from the living body.

The present invention was accomplished based on these findings. Also described herein are :

### (I) Methods for Diagnosing HCV Infection

(I-1) A method for diagnosing hepatitis C virus (HCV) infection, the method comprising a step of, taking leukocytes as a target, detecting an HCV protein present in the leukocytes.
(I-2) The diagnostic method according to (I-1), wherein the leukocyte is a monocyte.
(I-3) The diagnostic method according to (I-1) or (I-2), wherein the detection of an HCV protein is performed using an antibody to the protein.
(I-4) The diagnostic method according to (I-1) or (I-2), wherein the detection of an HCV protein is performed using a monoclonal antibody to an HCV core protein or HCV NS4 protein.

### (II) Preventive Agent and Prevention Method for HCV Infection

(II-1) A preventive agent for HCV infection, the agent comprising an antibody to an HCV protein as an active ingredient.
(II-2) The preventive agent according to (II-1), wherein the antibody specifically reacts to an HCV core protein or HCV NS4 protein.
(II-3) The preventive agent according to (II-1) or (II-2), wherein the antibody is a monoclonal antibody to an HCV protein.
(II-4) The preventive agent according to any of (II-1) to (II-3), the agent being a preparation for preventing HCV infection in a leukocyte.
(II-5) The preventive agent according to (II-4), wherein the leukocyte is a monocyte.
(II-6) A method for preventing HCV infection, the method comprising administering to a subject an effective dose of an antibody against an HCV protein.
(II-7) The prevention method according to (II-6), wherein the antibody specifically reacts to an HCV core protein or HCV NS4 protein.
(II-8) The prevention method according to (II-6) or (II-7), wherein the antibody is a monoclonal antibody to an HCV protein.
(II-9) Use of an antibody to an HCV protein for the preparation of a preventive agent for HCV infection.

### (III) Method for Treating HCV Infection

(III-1) A method for treating or ameliorating HCV infection, the method comprising a step of removing or reducing HCV-infected leukocytes from blood of an HCV-infected patient.
(III-2) The treatment or amelioration method according to (III-1), the method comprising the following steps (1) to (3):
   (1) a step of collecting blood from the body of an HCV-infected patient,
   (2) a step of removing HCV-infected leukocytes from the collected blood, and
   (3) a step of returning the blood from which leukocytes have been removed or reduced by step (2) to the body of the HCV-infected patient.
(III-3) The treatment or amelioration method according to (III-1) or (III-2), wherein the leukocyte is a monocyte.

The invention includes the following aspects:

### (IV) Methods for Screening Anti-HCV Drug Candidates

(IV-1) A method for screening an anti-HCV drug candidate, the method comprising the following steps (A) to (D):
   (A) a step of causing HCV to come into contact with non-HCV-infected monocytes in the presence of a test substance,
   (B) a step of measuring the HCV protein concentration in the monocytes obtained in step (A),
   (C) a step of comparing the HCV protein concentration measured in step (B) with the HCV protein concentration in control monocytes (control HCV protein concentration), which are caused to come into contact with HCV in the absence of the test substance, and
   (D) a step of selecting the test substance as an anti-HCV drug candidate when the HCV protein concentration measured in step (B) is lower than the control HCV protein concentration.
(IV-2) A method for screening an anti-HCV drug candidate, the method comprising the following steps (a) to (c):
   (a) a step of causing a test substance to come into contact with HCV-infected monocytes,
   (b) a step of measuring the HCV protein concentration (HCV protein concentration) in the monocytes obtained in step (a), and
   (c) a step of selecting the test substance as an anti-HCV drug candidate when the HCV protein concentration measured in step (b) is lower than the HCV protein concentration in the monocytes before the contact with the test substance, or when the HCV protein concentration measured in step (b) is lower than the HCV protein concentration in control HCV-infected monocytes which are not caused to come into contact with the test substance.
(IV-3) The screening method of (IV-1) or (IV-2), wherein an HCV protein concentration in monocytes is measured by an immunoassay using an antibody to the HCV protein.

### EFFECTS OF THE INVENTION

The present invention provides a method for screening an anti-HCV drug candidate, which can be used as an HCV-infection preventive drug or an HCV-infection therapeutic drug. The present invention makes it possible to obtain a novel HCV-infection preventive drug having an inhibitory action on HCV infection in leukocytes, particularly monocytes, which causes the onset of HCV infection in vivo, or enables a novel HCV-infection therapeutic drug having a reducing action on the amount of HCV antigens in monocytes.

### BEST MODE FOR CARRYING OUT THE INVENTION

### (I) Method for Diagnosing HCV Infection

The diagnostic method for HCV infection also described herein comprises, taking leukocytes of a subject as targets, detecting HCV proteins present in the target leukocytes.

HCV is a virus having a single-stranded, positive sense RNA with a full length of about 9.5 kb, and the genomic structure thereof consists of a 5'-non-coding region, a coding region, and a 3'-non-coding region. The coding region herein refers to the region encoding proteins comprising a total of about 3,000 amino acid residues, which consist of the structural protein region composed of a core protein and envelope proteins (E1, E2/NS1), and the non-structural protein region composed of NS2, NS3, NS4 and NS5.

The "HCV proteins" that are subject to detection refer to the above HCV proteins; however, the partial proteins thereof are also encompassed in addition to the full-length proteins. Examples of the partial proteins include structural proteins, such as a core protein, envelope proteins (E1, E2/NS1), etc., and non-structural proteins such as NS2, NS3, NS4, NS5, etc. Preferable proteins for detection are a core protein and non-structural proteins, with a core protein and NS4 protein being more preferable.

Leukocytes can generally be classified into granulocytes, monocytes, and lymphocytes. A preferable leukocyte subject for measurement in the method is a monocyte. Therefore, "leukocytes", if simply used hereinafter in the specification, include "monocytes" as a preferable embodiment.

Target leukocytes for the measurement may be those present in various tissues (e.g., internal organs such as the liver, heart, kidney, spleen, brain, lung, pancreas, uterus, ovary, bladder, etc.; tissues such as bone marrow, blood vessels, lymph nodes, nerves, skeletal muscles, membrana mucosa including oral cavities, digestive tract, eyeballs, internal ears, joints, skin, etc.); however, leukocytes in blood, especially peripheral blood, are desirable in view of the convenience in collection.

Detection of HCV proteins present in leukocytes, particularly in monocytes, can usually be performed in accordance with an immunoassay, which uses the antigen-antibody reaction between an HCV protein, preferably a human HCV protein and an antibody thereto. The antibody to the HCV protein (hereinafter simply referred to as an "anti-HCV antibody") is obtained by using HCV proteins as immunogens, and can be suitably selected from monoclonal antibodies having specificity to the HCV protein.

The HCV proteins used herein as immunogens include, in addition to natural HCV proteins derived from humans (full-length proteins), recombinant HCV proteins (full-length proteins) obtained by genetic engineering methods, and partial proteins having a partial structure of these HCV proteins. Examples of such partial proteins include, as described above, structural proteins, such as a core protein, envelope proteins (E1, E2/NS1), etc., and non-structural proteins such as NS2, NS3, NS4, NS5, etc. Preferable HCV proteins are a core protein and non-structural proteins, and more preferable are a core protein and NS4 protein.

A monoclonal antibody can be produced in accordance with a standard manner. For example, hybridoma cells are produced by fusing plasma cells (immunocytes) of a mammal immunized with immunogens (human HCV proteins) with myeloma tumor cells (myeloma cells) of a mammal, selecting a desired antibody-producing clone, and culturing the selected clone (see, e.g., Hanfland, P., Chem. Phys. Lipids, 15, 105 (1975) : Hanfland, P., Chem. Phys. Lipids, 10, 201 (1976) : Koscielak, J., Eur. J. Biochem., 37, 214 (1978), etc.). Methods employable for collecting a desired monoclonal antibody to a human HCV protein include a method in which hybridoma cells are cultured in accordance with a standard manner and the monoclonal antibody in the culture supernatant is collected; a method in which hybridoma cells are administered to a mammal compatible therewith to proliferate, after which the monoclonal antibodies in the ascites are collected; etc. The above-mentioned culture supernatant and the ascites can be used as crude antibody solutions without further treatment, or purified by a standard manner to be used as purified anti-HCV antibodies.

Specific examples of preferable monoclonal antibodies used in the diagnostic method include a monoclonal antibody to HCV core protein (Takahashi, K. et al., J. Gen. Virol., 73:667-672, 1992), and a monoclonal antibody to HCV NS4 protein (Hijikata, et al., Proc. Natl. Acad. Sci., USA, 90; 10773-10777, 1993). They are commercially available (e.g., Biogenesis Ltd., Poole, UK).

An immunoassay for leukocytes, preferably monocytes, can be specifically performed as follows.

When blood is used as a test sample, as described in Example 2, for example, monocytes are isolated from the blood using density gradient centrifugation, the isolated monocytes are applied to and fixed on a slide glass, etc., and an antigen-antibody reaction using an anti-HCV antibody occurs on the slide glass, taking the fixed monocyte as a specimen.

Alternatively, when various tissues (e.g., internal organs such as the liver, heart, kidney, spleen, brain, lung, pancreas, uterus, ovary, bladder, etc.; tissues such as including bone marrow, blood vessels, lymph nodes, nerves, skeletal muscles, membrana mucosa including oral cavity, digestive tract, eyeballs, internal ears, joints, skin, etc.) are used as test samples, an antigen-antibody reaction using anti-HCV antibodies can be performed on leukocytes (monocytes) in these tissues. More specifically, as described in Example 1, an antigen-antibody reaction occurs using an anti-HCV antibody, taking formalin-fixed samples or cells of these tissues as specimens, and the leukocytes present in the tissues are stained to immunohistologically and specifically detect the HCV proteins present therein. In this case, the fixed samples of internal organs are, before use, usually deparaffinized in xylene, dehydrated in alcohol, and the endogenous peroxidase is inactivated with hydrogen peroxide.

The immunoassay can be performed in accordance with an RIA method, ELISA method, or a condensation method, etc. using a competition technique or sandwich technique, etc. The preferable method is the ELISA using a sandwich technique. Any indirect method, direct method, or high-sensitivity method (e.g., an LSAB method which employs a biotin-streptavidin reaction) can be used. The operations and procedures of these methods can follow those employed in a standard manner.

In the above case, the immunoassay can be more specifically performed by adding an anti-HCV antibody (preferably a mouse monoclonal antibody to HCV core protein or HCV NS4 protein) as a primary antibody to a slide glass on which leukocytes (monocytes) are applied and fixed or to fixed samples or cells of various organs for a reaction (antigen-antibody reaction), the reactant being subsequently reacted with a labeled secondary antibody, and further reacted with a chromophoric substrate which reacts with the labeling agent of the secondary antibody. In this case, the presence of HCV proteins in leukocytes (monocytes) can be detected by the leukocytes (monocytes) which are specifically stained.

Known secondary antibodies may be used as labeled secondary antibodies, and an anti-immunoglobulin antibody labeled with an enzyme is commonly used. The secondary antibodies usually used are anti-immunoglobulin antibodies obtained by immunizing animals such as mice, rats, guinea pigs, rabbits, sheep, goats, horses, cows, etc.; however, it is desirable that a secondary antibody is an antibody obtained by immunizing an animal different from the animal used to produce the primary antibody (anti-HCV antibody). The enzyme used to label the secondary antibody is not limited, and examples include peroxidases such as horseradish peroxidase; alkaline phosphatase; β-galactosidase, acid phosphatase, etc., with peroxidase being preferable.

The chromophoric substrate is not limited insofar as it reacts with an enzyme used to label a secondary antibody, and can be suitably selected for use. When using peroxidase as an enzyme, for example, it is not limited, o-phenylenediamine (OPD) and DAB (diaminobenzidine) are usable. The color reaction can be terminated by a standard method, and an example is a method in which a suitable enzyme activity inhibitor, such as 1 to 4N sulfuric acid, etc., is added to a reaction solution.

Immunoreaction conditions for the immunoassay are not limited, and the same conditions as commonly employed in this type of assay can be used. For example, any typical solvents which do not adversely affect the reaction can be used for the above measurement system, and examples include buffer solutions whose pH is about 5.0 to about 9.0, such as citrate buffer, phosphate buffer, physiological saline-containing phosphate buffer (PBS), trishydrochloric acid buffer, acetate buffer, etc.

Alternatively, the method for diagnosing HCV infection can be performed by a 3-step sandwich immunoassay using an anti-HCV antibody. This method is, for example, usually performed as follows. An antibody to leukocytes is solid-phased on a suitable carrier, such as a 96-well plate, as a primary antibody. The blood of a subject is allowed to stand for a reaction to the solid-phased antibody overnight at room temperature (Step 1). Subsequently, an anti-HCV antibody (HCV monoclonal antibody) as a secondary antibody is added to the above carrier (96-well plate) and reacted at room temperature for about 2 hours, thereby reacting the secondary antibody with the reactant (a complex between the anti-leukocyte antibody and the leukocytes) obtained by Step 1 (Step 2). A certain amount of a labeled antibody, such as an enzyme-labeled anti-rabbit IgG antibody, etc., is further reacted with the reactant (a reacted complex between the anti-HCV antibody and the leukocytes) obtained by Step 2 mentioned above at room temperature for about 2 hours (Step 3). Unbound labeled antibodies are isolated and removed from the conjugate between the reacted complex and the labeled antibody obtained in Step 3 above, to which a coloring solution is added to initiate a color reaction. The color reaction is then terminated using 2N sulfuric acid, thereby measuring the absorbance of the obtained color-reacted solution. The HCV-infected leukocytes are thus detected and assayed.

The presence of HCV infection in vivo can be diagnosed by detecting the presence of HCV proteins in the leukocytes, i.e., the presence of HCV infection in the leukocytes, of a subject. More specifically, when HCV proteins are detected in the leukocytes, particularly in the monocytes, of a subject, the subject is diagnosed as being infected with HCV. The diagnostic method uses direct detection to determine the presence of the infection at an earlier stage without having to wait for the antibody production. Further, the present method makes it possible to observe the effects and prognostic development of drug treatments in HCV-infected patients in addition to the diagnosis of HCV infection. As mentioned earlier, since the diagnostic method directly measures the HCV proteins present in leukocytes, it is a more reliable diagnostic method than conventional HCV infection diagnostic methods, which use anti-HCV antibodies as indicators that may remain in the body after the treatment.

### (II) Preventive Agent for HCV Infection

As shown in Example 3, when the serum of an HCV-infected patient is caused to come into contact with the leukocytes (monocytes) of a non-HCV-infected healthy subject, HCV infects the leukocytes. However, if antibodies (anti-HCV antibodies) to the HCV proteins are present in the system, the above-mentioned HCV infection in leukocytes can be inhibited. Further, as shown in Example 5 (2), interferon-α (IFN-α), a known anti-HCV drug, was verified to have an equivalent inhibitory action on HCV infection in leukocytes. Further, as shown in Example 4, when HCV-infected leukocytes (monocytes) are cultivated with the known anti-HCV drug interferon-α (IFN-α), the amount of HCV antigens in the leukocytes is reduced.

Based on these findings, it is presumed that HCV infection in leukocytes, particularly monocytes, is associated with or triggers HCV infection in vivo, and that HCV infection in vivo can be blocked (prevented) by inhibiting HCV infection in leukocytes. Namely, a substance having an inhibitory action on HCV infection in leukocytes, e.g., an anti-HCV antibody, etc., is useful as an active ingredient of a preventive agent for HCV infection.

Accordingly, a preventive agent for HCV infection that contains an anti-HCV antibody as an active ingredient is described herein.

As explained in (I) above, anti-HCV antibodies include those to full-length proteins of human HCV, and those to partial proteins thereof. Examples of the antibodies to such partial proteins include those to structural proteins such as a core protein, envelope proteins (E1, E2/NS1), etc., and those to non-structural proteins such as NS2, NS3, NS4, NS5 proteins, etc. Preferable are antibodies to a core protein or antibodies to non-structural proteins, and more preferable are antibodies to a core protein and antibodies to NS4 protein.

A monoclonal antibody is preferable due to its specificity to an HCV protein, and a human antibody having low antigenicity to humans, a humanized antibody (CDR-grafting antibody) or a chimeric antibody is even more preferable in view of applicability to humans. The production processes of these antibodies are known, and the antibody can also be prepared in accordance with standard methods (e.g., Eda et al., J. Virol., 80:5552-5562, 2006, etc.).

The preventive agent for HCV infection requires containing such anti-HCV antibodies as active ingredients, and other components may or may not be contained as long as these antibodies are contained. For example, the preventive agent for HCV infection may contain a pharmaceutically acceptable carrier or additive, in addition to an effective amount of anti-HCV antibodies.

The effective amount of anti-HCV antibodies herein is not limited insofar as the amount is capable of inhibiting HCV infection in leukocytes. A typical example of a minimal effective amount of anti-HCV antibodies to 1 µg of HCV proteins is 1 ng. The amount of anti-HCV antibodies contained in the preventive agent for HCV infection can be determined based on this amount. For example, it is desirable that the preventive agent contain anti-HCV antibodies so that the amount of anti-HCV antibodies in a single dose for a subject (adult) is 0.1 mg to 1 g, and preferably 1 to 100 mg.

Examples of pharmacologically acceptable carriers contained in the above preventive agent for HCV infection include a wide variety of products commonly used in the relevant field in accordance with various administration forms to be described later. Examples include fillers, expanders, binders, humectants, disintegrants, surface-active agents, lubricants, buffers, tonicity agents, chelating agents, pH adjusting agents, surfactants, etc. Further, the preventive agent for HCV infection may also contain, as necessary, stabilizers, disinfectants, coloring agents, preservatives, essence, flavoring agents, sweeteners, etc.

The preventive agent for HCV infection can be administered orally or parenterally, and can be prepared in pharmaceutical formulations appropriate to these administration routes (e.g., powders, granules, tablets, pills, capsules, syrups, emulsions, elixirs, suspensions, solutions, injection solutions, drops, suppositories, etc.).

The parenteral administrations herein include subcutaneous injection, intravenous injection, intramuscular injection, intraperitoneal injection, drip, or the like. Preparations for injections, such as aqueous suspensions and oily suspensions for sterile injections, can be prepared by a known process in the relevant field, using a suitable dispersant or humectant and suspending agent. Such preparations for sterile injections may be, for example, sterile injection solutions or suspensions among parenterally-administrable diluents or solutions acceptable for the preparation, such as aqueous solutions, etc. Acceptable vehicles or solutions for use include water, Ringer solution, isotonic sodium chloride solution, etc. Further, sterile nonvolatile oils or fatty acids can be typically used as a solvent or slurrying solvent. Examples of nonvolatile oils and fatty acids include natural, synthetic or semi-synthetic fatty oils or fatty acids as well as natural, synthetic or semi-synthetic mono-, di- or triglycerides.

Suppositories for rectal administration can be prepared by mixing medicinal substances therein and a suitable low-stimulant excipient, such as cocoa butter and polyethylene glycols which are solid at room temperature but melt to a liquid at intestinal temperatures to release the medicinal substances.

Examples of solid forms for oral administration include the above-mentioned powders (pulverizates), granules, tablets, pills, capsules, etc. In these forms, anti-HCV antibodies as active components may be mixed with at least one additive, such as sucrose, lactose, cellulose sugar, mannitol, maltitol, dextran, starches, agars, alginates, chitins, chitosans, pectins, tragacanth gums, gum arabics, gelatins, collagens, casein, albumin, synthesized or semi-synthesized polymers, or glycerides. The preparations of such forms may further contain other additives as in typical forms. Examples of other additives include lubricants such as inactive diluents, magnesium stearate, etc.; preservatives such as parabens, sorbic acids, etc.; antioxidants such as ascorbic acids, α-tocopherols, cysteines, etc.; disintegrants, binders, thickeners, buffering agents, sweeteners, flavoring agents, perfumes, etc. Tablets and pills may further be enterically coated.

Examples of solutions for oral administration include pharmaceutically acceptable syrups, emulsions, elixirs, suspensions, solutions, etc. These solutions may contain inactive diluents, such as water, commonly used in the relevant field.

The dose of the preventive agent for HCV infection can be suitably determined according to the age, weight, health condition, gender, and diet of a subject, the administration time, the administration method, the frequency of excretion, the degree of the disease being treated at the time of taking the preventive agent, etc.

As described earlier, a typical example of the minimal effective amount of anti-HCV antibodies to 1 µg of HCV proteins is 1 ng. Accordingly, a single dose of the preventive agent for HCV infection can be selected on the basis of the amount given above. For example, the preventive agent for HCV infection can be administered in an amount so that the amount of anti-HCV antibodies in a single dose per subject (adult) is 0.1 mg to 1 g, and preferably 1 to 100 mg.

Further, a method for preventing HCV infection is described. The method comprises administering to a subject a substance having an action which inhibits HCV infection in leukocytes. Preferable examples of HCV-infection inhibitory substances include the anti-HCV antibodies mentioned earlier. More specifically, the method for preventing HCV infection can be conducted by administering to a subject anti-HCV antibodies, particularly the preventive agent for HCV infection described earlier. The dose is as mentioned earlier.

### (III) Method for Treating or Ameliorating HCV Infection

Further, a method for treating or ameliorating HCV inflection is described

Infectious diseases with hepatitis C virus (HCV infectious diseases) are not limited to liver illnesses such as hepatitis C, but encompass other diseases triggered by hepatitis C virus infection. More specifically, the HCV infectious diseases include, for example, hepatitis caused by hepatitis C virus (hepatitis C), liver cirrhosis, hepatoma, myocarditis, heart failure, nephritis, renal failure, cystitis, myositis, pancreatitis, Sjogren's syndrome, lymphoma, porphyria cutanea tarda, lichen planus, arthritis, diabetes, arteriosclerosis, angitis, and cranial nerve diseases.

The method for treating or ameliorating HCV infection as described herein can be basically conducted by removing or reducing the leukocytes infected with hepatitis C virus from the blood of an HCV-infected patient (leukocytapheresis therapy or leukocyte reduction therapy).

Leukocytapheresis therapy (LCAP therapy) is the blood purification procedure conventionally employed to treat ulcerative colitis or rheumatoid arthritis, and it can also be used in the method for treating or ameliorating HCV infection
The method essentially comprises the following steps (1) to (3);
(1) a step of collecting blood from the body of an HCV-infected patient,
(2) a step of removing leukocytes infected with HCV from the collected blood, and
(3) a step of returning the blood cleansed in step (2) to the body of the HCV-infected patient.

More specifically, the method is conducted by collecting blood to a location outside the body from the vein of an elbow or a thigh, removing the targeted leukocytes using a leukocyte removing apparatus or specific gravity differences, and returning the cleansed blood to a vein on the opposite side of the body.

The leukocyte removing apparatus herein is not limited, and examples include a column filled with leukocyte-adsorbing beads, and an apparatus filled with leukocyte-adsorbing fibers or filters (Cellsorba). An example of a leukocyte-adsorbing bead is a cellulose acetate bead which has a good ability to adsorb granulocytes and monocytes among the leukocytes (G-1 bead, JIMRO Co., Ltd., Japan). The leukocyte-adsorbing fibers and filters are not limited so long as they are made from microfibers of 3 µm or less in length, because leukocytes exhibit an adhering property to such microfibers, and specific examples include microfibers made of polyesters, etc., or filters composed of such microfibers (e.g., Sepacell RZ, Asahikasei Medical Co., Ltd. Japan; Purecell RC RC400H, Pall Corporation, USA; IMUGARD III-RC, Terumo Corporation, Japan, etc.)

In addition to the use of leukocyte removing apparatuses, leukocytes (monocytes) can be separated and removed by a centrifugation method, using specific gravity differences among blood components.

Further, usable methods for reducing the leukocyte count (leukocyte reduction therapy) include those in which known leukocyte-reducing drugs, such as anti-cancer agents, antitumor drugs and leukemia therapeutic drugs are administered. Leukocyte reduction therapy by the administration of these drugs can be performed in combination with the leukocytapheresis therapy described above.

When leukocyte-forming cells in the bone marrow are infected with HCV, HCV-infected leukocytes may be formed again and released into the blood even after the leukocytapheresis therapy. In this case, it is desirable that anti-virus therapies such as interferon administration be continued after the leukocytapheresis therapy.

### (IV) Method for Screening Anti-HCV Drug Candidates

### (IV-1) Method for Screening Preventive Drug Candidates for HCV Infection

The results of Examples 3 and 5 (2) show, as described earlier, that HCV infection in leukocytes, particularly in monocytes, is related to HCV infection in vivo, and HCV infection in vivo can be blocked (prevented) by arresting HCV infection in leukocytes. Namely, a substance having an inhibitory action on HCV infection in leukocytes is useful as an anti-HCV drug, particularly as a preventive agent for HCV infection.

Based on these findings, an anti-HCV drug having an inhibitory action on HCV infection, i.e., a preventive drug for HCV infection, can be screened by using an inhibitory action on HCV infection in leukocytes as an indicator.

The screening can be performed by the following steps (A) to (D):
(A) a step of causing hepatitis C virus to come into contact with non-HCV-infected monocytes in the presence of a test substance,
(B) a step of measuring the concentration of hepatitis C virus proteins in the monocytes (HCV protein concentration) obtained in step (A),
(C) a step of comparing the HCV protein concentration measured in step (B) and the HCV protein concentration in control monocytes which are caused to come into contact with hepatitis C virus in the absence of the test substance (control HCV protein concentration), and
(D) a step of selecting the test substance as an anti-HCV drug candidate when the HCV protein concentration measured in step (B) is lower than the control HCV protein concentration.

The test substance used herein may be either a nucleic acid (including polynucleotides), a peptide (including polypeptides), an organic compound, or an inorganic compound. The screening can be performed by causing hepatitis C virus to come into contact with non-HCV-infected monocytes in the presence of such a test substance or a sample containing such a test substance (test sample). Examples of the test sample include cell extracts, expression products of gene libraries, extracts from natural products such as animals or plants, etc.

Hepatitis C virus (HCV) is preferably a human HCV which infects humans. HCV caused to come into contact with monocytes does not have to be a purified product as long as leukocytes are not mixed therein, and any HCV-containing product, such as a serum from an HCV-infected patient, etc., can be used. The monocyte that is to be brought into contact with an HCV must be one not infected with HCV, and examples include a monocyte or cultured monocyte derived from a healthy, non-HCV-infected subject (e.g., U937 cell line used in Example 5).

Employable conditions under which HCV is caused to come into contact with monocytes in step (A) may be those wherein HCV and monocytes do not die, and a typical example includes that according to an in vivo environment. The conditions are not limited, and preferable are conditions which use, for example, RPMI medium supplemented with 10% fetal calf serum at 37°C in the presence of 5% carbon dioxide gas as employed in examples to be described later.

The measurement of HCV protein concentration in monocytes in step (B) can be performed by an immunoassay using antibodies to HCV proteins. The details are as explained in (I), and an ELISA using sandwich method is preferably used.

It can be confirmed that the test substance selected in step (D) is a substance that is able to inhibit HCV infection in monocytes when HCV is caused to come into contact therewith.

As described earlier, HCV infection in vivo develops when HCV infects leukocytes, particularly monocytes. Thus, the above substance with an ability to inhibit HCV infection in monocytes can block (prevent) HCV infection in vivo. Consequently, the specimen selected by the above-mentioned screening method of the present invention can be an anti-HCV drug, particularly a preventive drug for HCV infection.

### (IV-2) Method for Screening HCV Therapeutic Drug Candidates

Example 4 shows that known HCV therapeutic drug IFN-α acts to reduce the amount of HCV antigens in leukocytes, particularly in monocytes. This finding suggests that a substance that reduces the amount of HCV antigens in leukocytes, particularly in monocytes, is useful as an anti-HCV drug, particularly as an HCV therapeutic drug.

Based on this suggestion, an anti-HCV drug that acts to treat HCV infection, i.e., an HCV therapeutic drug, can be screened by using, as an index, a reduced amount of HCV antigens (HCV protein concentration) in leukocytes, particularly in monocytes.

The screening has the following steps (a) to (c):
(a) a step of causing a test substance to come into contact with hepatitis C virus-infected monocytes,
(b) a step of measuring the concentration of hepatitis C virus proteins (HCV proteins) in the monocytes obtained in step (a), and
(c) a step of selecting the test substance as an anti-HCV drug candidate when the HCV protein concentration measured in step (b) is reduced from the HCV protein concentration in the monocytes before coming into contact with the test substance, or lower than the HCV protein concentration in control HCV-infected monocytes, which are not caused to come into contact with the test substance.

Examples of the specimen include those described in (IV-1) above. The monocyte caused to come into contact with a specimen does not have to be a purified product, and the blood collected from a patient infected with HCV may be used.

Employable conditions under which HCV is caused to come into contact with monocytes in step (a) may be those wherein HCV and monocytes do not die, as in the method described in (IV-1) , and an example includes conditions according to an in vivo environment. These conditions are not limited, and preferable conditions are those which use, for example, RPMI medium supplemented with 10% fetal calf serum at 37°C in the presence of 5% carbon dioxide gas as employed in examples to be described later.

The measurement of the HCV protein concentration in monocytes in step (b) can be performed, as in the method described in (IV-1), by an immunoassay that uses antibodies to HCV proteins. The details are as explained in (I), and an ELISA sandwich method is preferably used.

It can be determined that the test substance selected in step (c) is a substance that is able to reduce the amount of HCV antigens in leukocytes (monocytes) infected with HCV. As mentioned earlier, since a substance that is able to reduce the amount of HCV antigens in monocytes is useful as an HCV therapeutic drug, the test substance selected by the above screening method can be an anti-HCV drug, in particular an HCV therapeutic drug.

The anti-HCV drug candidate thus selected by methods (IV-1) and (IV-2) above may be further subjected to drug efficacy tests, safety tests, and clinical trials on patients infected with HCV, and a more practical anti-HCV drug as a preventive agent or therapeutic agent for HCV infection can be obtained by performing these tests.

The anti-HCV drug candidate thus selected, based on further results of structural analysis, can be industrially produced by chemical synthesis, biological synthesis (fermentation), or gene manipulation for the use as an anti-HCV drug that is effective in preventing or treating HCV infection.

### Examples

### Reference Example 1 Detection of HCV Antigens in Leukocytes

(1) Formalin-fixed tissue samples (sections having a 5-µm thickness) of liver, heart, kidney, aorta, lymph node, and bone marrow obtained from HCV-infected patients were deparaffinized using xylene, dehydrated with alcohol, and then treated with hydrogen peroxide to inactivate endogenous peroxidase. The target HCV-infected patients herein are those whose sera were anti-HCV antibody positive.
   Using each of the obtained tissue samples as a specimen, 0.2 mL (20 µg/mL) of a mouse monoclonal antibody to the HCV core region protein (Institute of Immunology Co., Ltd., Core A-2 antibody, Takahashi, K. et al., J. Gen. Virol., 73:667-672, 1992, hereinafter referred to as "core antibody") or a mouse monoclonal antibody to the NS4 protein in the HCV non-structural region (obtained from The Research Institute for Microbial Diseases of Osaka University, Kanonji Institute, Research Group), NS4a antibody, Hijikata et al., Proc. Natl. Acad. Sci., USA., 90, 10773-10777, (1993), hereinafter referred to as "NS4 antibody") were respectively added to each specimen as a primary antibody, and incubated overnight at 4°C. Subsequently, immunostaining was performed using a VECTASTAIN ABC mouse IgG kit (Burlingame, CA, USA), according to the manufacturer's protocols, and DAB (diaminobenzidine) was developed.

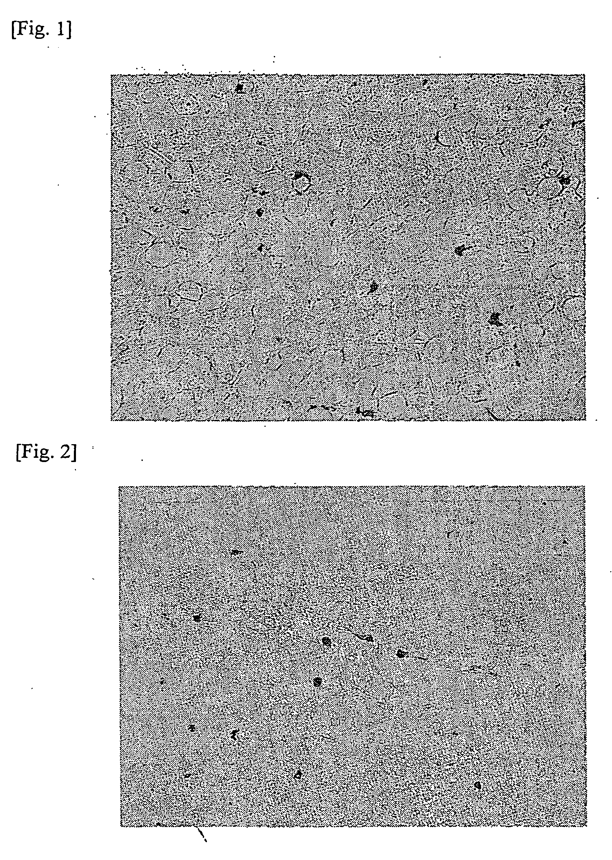

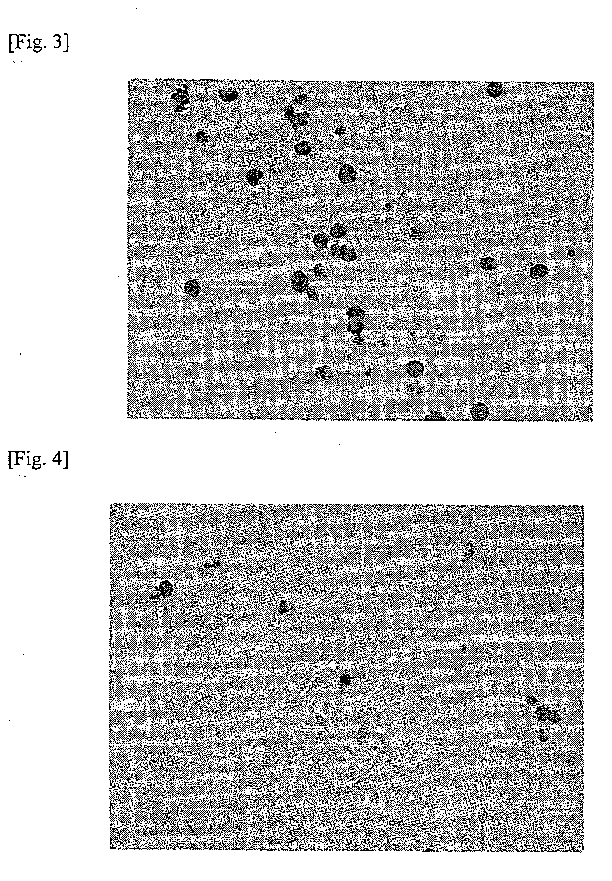

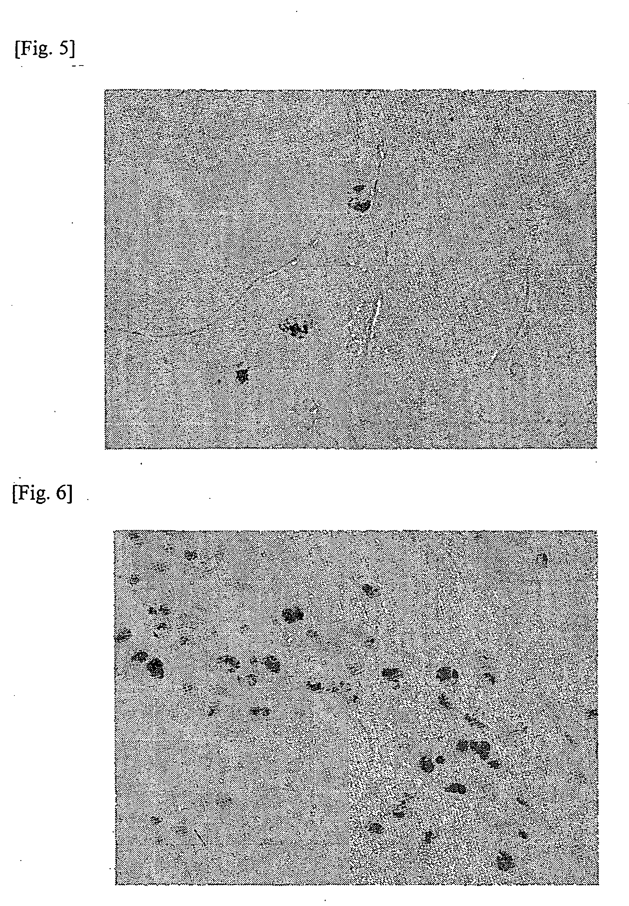

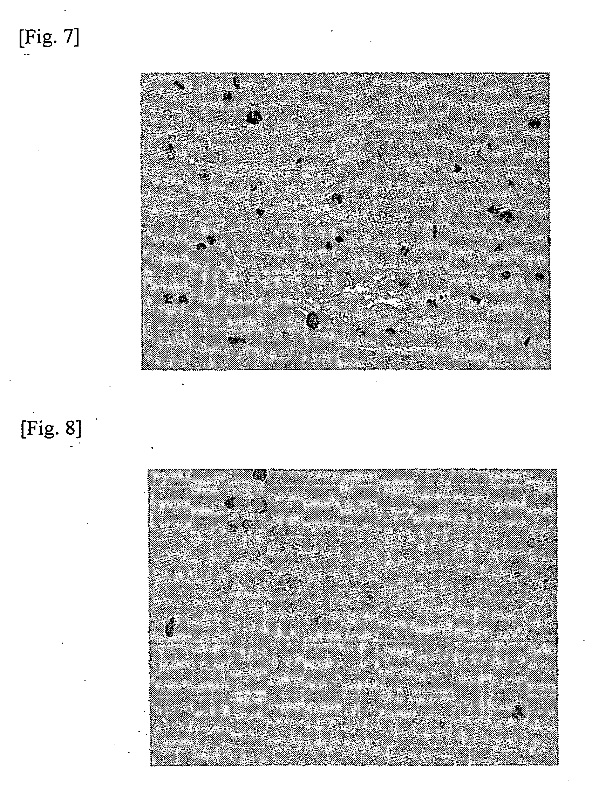

   Figures 1 to 7 show the obtained results. Figure 1 is a microscopic image of hepatic tissues reacted with the NS4 antibody. Figure 2 is a microscopic image of cardiac tissues reacted with the NS4 antibody. Figure 3 is a microscopic image of hepatic tissues reacted with the core antibody. Figure 4 is a microscopic image of cardiac tissues reacted with the core antibody. Figure 5 is a microscopic image of renal tissues reacted with the core antibody. Figure 6 is a microscopic image of aortic tissues reacted with the core antibody. Figure 7 is a microscopic image of lymph node tissues reacted with the core antibody, and Fig. 8 is a microscopic image of bone marrow tissues reacted with the core antibody.
   As shown in the figures, it is verified that the leukocytes were stained in each of the tissues of liver (Figs. 1 and 3), heart (Figs. 2 and 4), kidney (Fig. 5), aorta (Fig. 6), lymph node (Fig. 7), and bone marrow (Fig. 8) from the HCV-infected patients. This finding reveals that HCV antigens are localized in the leukocytes of HCV-infected patients.
(2) Further, to study the specificity of these immunoreactions, the following experiment was conducted in the same manner as in (1) above, using hepatic tissue samples from HCV-infected patients.

20 µg/mL of the core antibody as a primary antibody and 10 µg/mL of the core antigen (HCV core protein) used as the immune source therefor were admixed with each of the hepatic tissue samples, which were treated to inactivate peroxidase therein in the same manner as in (1) above. The mixture was reacted at room temperature for 2 hours. Subsequently, immunostaining (DAB development) was performed using a VECTASTAIN ABC mouse IgG kit (Burlingame, CA, USA), according to the manufacturer's protocols. Further, as a control test, hepatic tissue samples from HCV-infected patients reacted with 20 µg/mL of the core antibody without adding the core antigen (HCV core protein) were subjected to immunostaining in the same manner as described above.

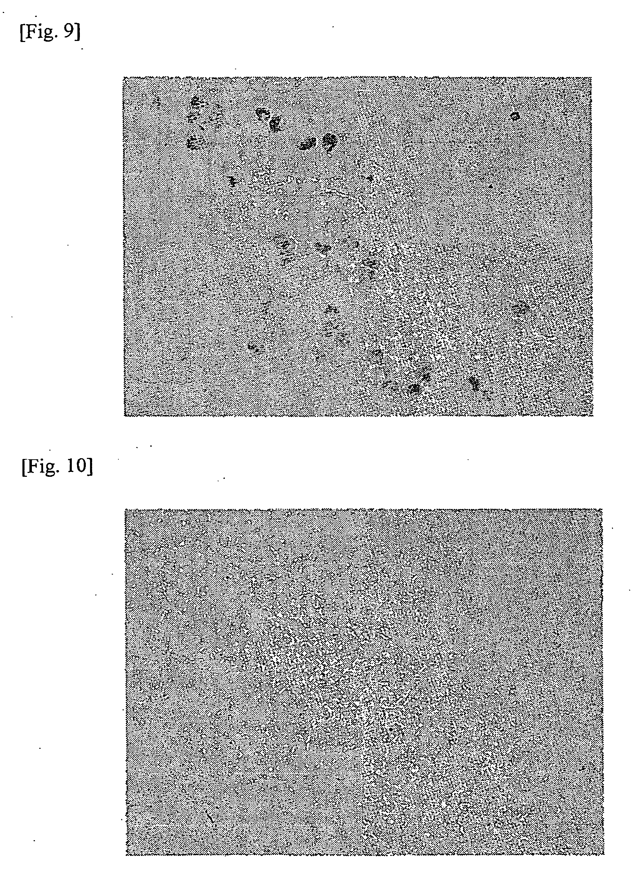

The results are shown in Figs. 9 and 10. Figure 9 shows a microscopic image of the control hepatic tissues reacted with the core antibody without adding the core antigen (control test), and Fig. 10 shows a microscopic image of the hepatic tissues reacted with the core antibody that had been reacted with the core antigen beforehand.

The staining of leukocytes was observed in the control test (Fig. 9), thereby verifying the localization of the HCV core antigen in leukocytes of the hepatic tissues. However, in Fig. 10, the positive image shown in Fig. 9 was not observed. The reason for this is presumably that the core antibody and the core antigen formed a complex by the antigen-antibody reaction, and failed to react with the HCV core antigen present in leukocytes of the hepatic tissues. This indicates that the core antibody specifically reacts with the HCV core antigen present in leukocytes.

Moreover, using heart, kidney, aorta, lymph node, and bone marrow tissue samples, the same test as performed on the hepatic tissues described above was conducted, and the specific reaction of the core antibody was also evident in these tissue samples.

### Reference Example 2 Detection of HCV Antigens in Peripheral Mononuclear Blood Cells

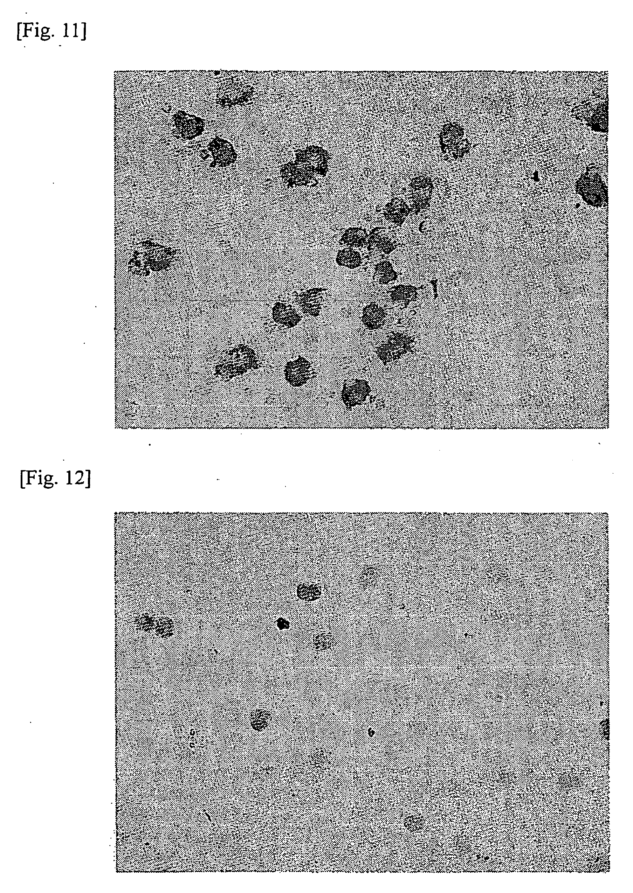

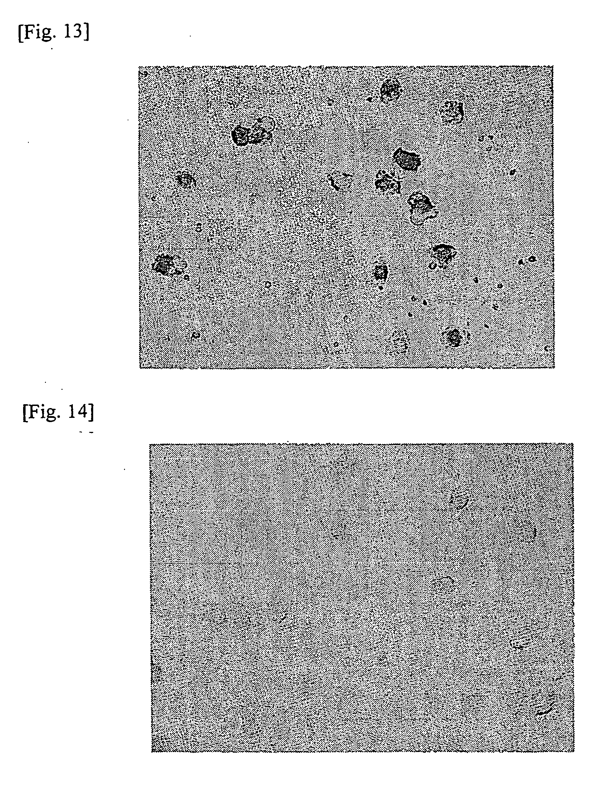

(1) Peripheral blood was collected from HCV-infected patients and non-HCV-infected healthy subjects, respectively, and monocytes of the leukocytes were isolated from the collected blood by density gradient centrifugation using Ficoll pack solution (Amersham, USA). The isolated monocytes were applied to a slide glass, dried, and fixed with ethanol. Using such monocytes as specimens, immunostaining was performed in the same manner as in Example 1. More specifically, 0.2 mL (20 µg/mL) of the core antibody or the NS4 antibody was added to monocytes of HCV-infected patients and non-HCV-infected subjects, and incubated overnight at 4°C. Subsequently, immunostaining was performed using a VECTASTAIN ABC mouse IgG kit for the DAB development.
   The results are shown in Figs. 11 to 14. Figures 11 and 12 are, respectively, a microscopic image showing the reaction result of monocytes from HCV-infected patients with the NS4 antibody, and the reaction result of monocytes from non-HCV-infected subjects with the NS4 antibody. Further, Figs. 13 and 14 are, respectively, a microscopic image showing the reaction result of monocytes from HCV-infected patients with the core antibody, and the reaction result of monocytes from non-HCV-infected subjects with the core antibody. As shown in Figs. 11 and 13, monocytes from HCV-infected patients react with both the NS4 antibody and the core antibody, i.e., HCV antibodies, whereby the immunostained images were observed. In contrast, such immunostained images were not observed with monocytes from non-HCV-infected subjects, as shown in Figs. 12 and 14.
(2) Subsequently, serum collected from an HCV-infected patient (HCV quantitative value: 225 KIU/mL) was added to monocytes (1 x 10⁶ cells/mL) from a non-HCV-infected subject, and incubated for 96 hours in RPMI1640 medium supplemented with 10% fetal calf serum (BSA). After incubation, the obtained culture was reacted to the NS4 antibody for immunostaining in the same manner as in Example 1. More specifically, 0.2 mL (20 µg/mL) of the NS4 antibody was added to the culture, incubated overnight at 4°C, and immunostaining was performed using a VECTASTAIN ABC mouse IgG kit, whereby DAB was developed. In addition, as a control test, monocytes (1x 10⁶ cells/mL) from a non-HCV-infected subject were incubated in the same manner using serum from a non-HCV-infected subject, in place of the serum collected from the HCV-infected patient described above (HCV quantitative value: 225 KIU/mL), followed by reacting the culture to the NS4 antibody for the immunostaining.

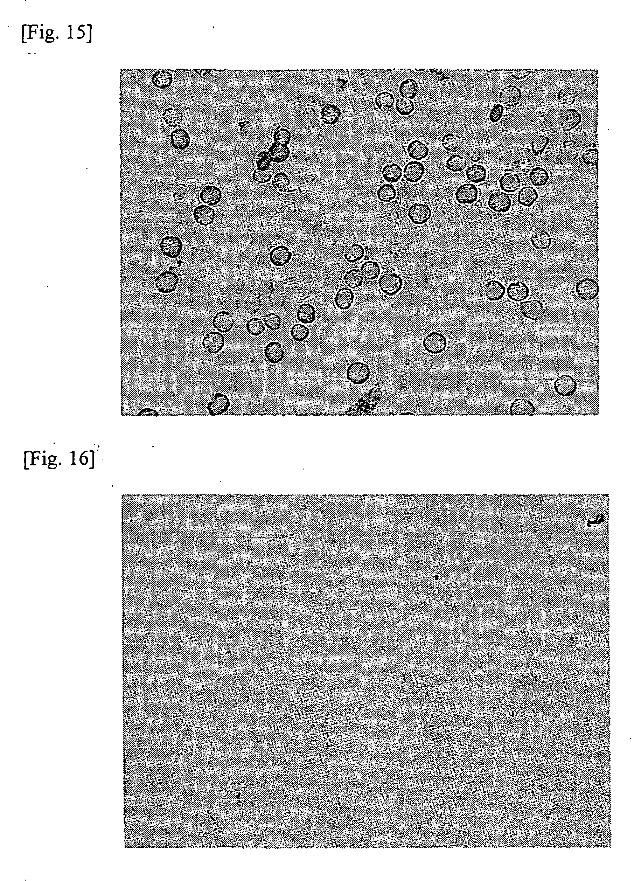

Figure 15 shows a microscopic image of the monocytes reacted to the serum of the HCV-infected patient, and Fig. 16 shows a microscopic image of the monocytes reacted to the serum of the non-HCV-infected subject. As shown in the figures, the immunostained image was not observed when the serum of the non HCV-infected subject was added to the monocytes of the non-HCV-infected subject (Fig. 16). However, the immunostained image was observed in the monocytes when the serum derived from the HCV-infected patient was added to the monocytes of the non-HCV-infected subject (Fig. 15), as observed in the HCV-infected patient (see Figs. 11 and 13).

The above results (1) and (2) revealed that HCV infects monocytes of leukocytes. Based on this finding, it is verified that HCV infection can be diagnosed, using monocytes as targets, by detecting the presence of HCV antigens in monocytes.

### Reference Example 3 Prevention and Treatment of HCV Infection

10 µL each of (1) 10 µg/mL of the core antibody (the same one described in Example 1), (2) 60 µg/mL of the NS4 antibody (the same one described in Example 1), and, as a control, (3) 10 µg/mL of mouse IgG (Dako) was added to and mixed with 10 µL of serum collected from a patient infected with HCV (HCV quantitative value: 225 KIU/mL) diluted 100 times using a phosphate buffer, and each mixture was incubated overnight at 4°C.

Separately, peripheral mononuclear cells were isolated from a non-HCV-infected subject, adjusted to give 1 x 10⁶ cells/mL using RPMI medium supplemented with 10% fetal calf serum, and a quantity of 500 µL of the obtained suspension was pipetted into each well of a 24-well plate.

20 µL of a mixture consisting of 10 µL each of (1) to (3) above and 10 µL of the above serum from the HCV-infected patient (dilution) were added to each well of the plate, and the mixture was incubated for 4 days at 37°C in the presence of 5% carbon dioxide gas. The cultured monocytes were then collected from each well, and subjected to immunostaining using the core antibody in the same manner as in Example 1.

The obtained results are shown in Figs. 17 to 19. Figure 17 is a microscopic image showing the immunostaining result of the non-HCV-infected subject's monocytes reacted to the mixture of the HCV-infected patient's serum and (1) the core antibody. Figure 18 is a microscopic image showing the immunostaining result of the non-HCV-infected subject's monocytes reacted to the mixture of the HCV-infected patient's serum and (2) the NS4 antibody, and Fig. 19 is a microscopic image showing the immunostaining result of the non-HCV-infected subject's monocytes reacted to the mixture of the HCV-infected patient's serum and (3) mouse IgG.

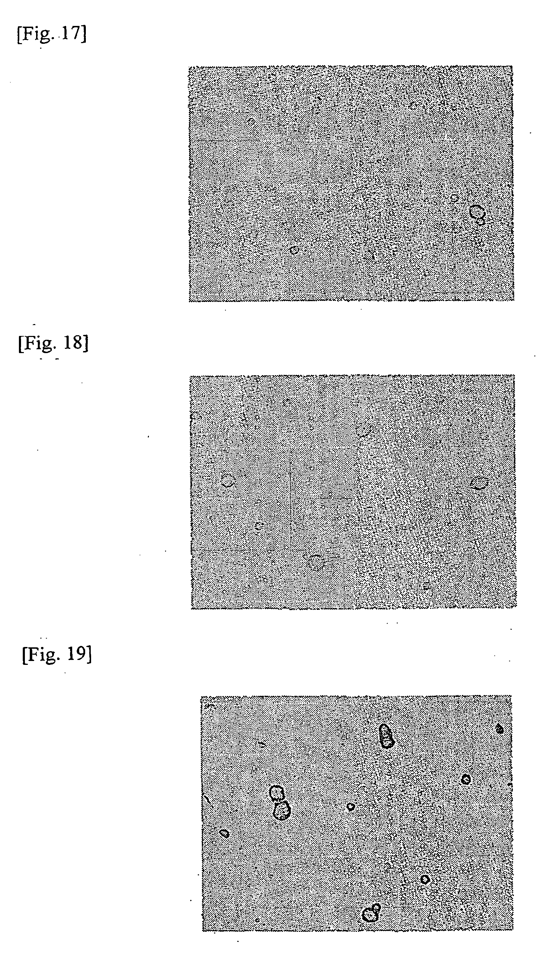

As shown in Figs. 17 and 18, when (1) the core antibody or (2) the NS4 antibody is mixed in the serum of an HCV-infected patient, the stain intensity of a non-HCV-infected subject's monocytes was evidently diminished, in comparison with the case wherein (3) mouse IgG was mixed into the serum from an HCV-infected patient (Fig. 19). This means that HCV infection in monocytes which supposedly develops by mixing monocytes with the serum of an HCV-infected patient (introduction of HCV antigens to monocytes) is suppressed by the presence of the core antibody and the NS4 antibody, i.e., HCV antibodies, and more specifically, HCV infection can be blocked (prevented) by HCV antibodies (core antibody or NS4 antibody).

### Example 4 Evaluation of an Anti-Virus Drug that Uses HCV-infected Peripheral Mononuclear Cells

Peripheral mononuclear cells were isolated from an HCV-infected patient in the same manner as in Example 3, and 1 x 10⁵ µg/mL of human interferon-α (human IFN-α) (PeproTech EC, London, UK), known HCV therapeutic drug, was added thereto, followed by incubation for 5 days. Further, as a comparative experiment, peripheral mononuclear cells from an HCV-infected patient were similarly incubated for 5 days without adding human IFN-α. Subsequently, each of the cultured monocytes was collected, and immunostaining was performed in the same manner as in Example 1, using the core antibody as the primary antibody.

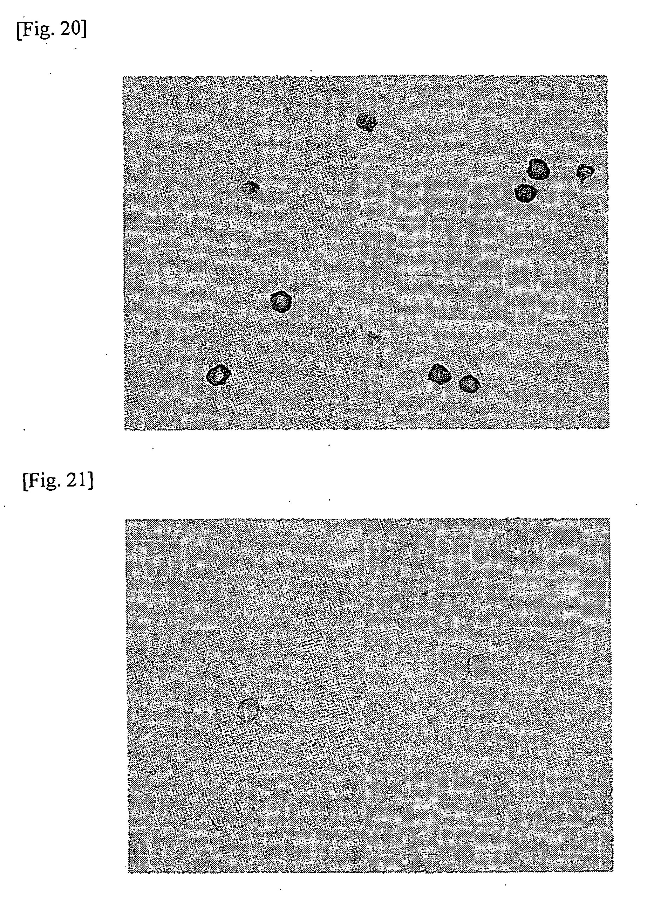

Figure 20 shows a microscopic image of the immunostaining result of the monocytes incubated without adding human IFN-α, and Fig. 21 shows a microscopic image of the immunostaining result of the monocytes incubated with human IFN-α added thereto. The results shown in Figs. 20 and 21 revealed that when INF-α was added to the monocytes of an HCV-infected patient, the immunostain intensity of the non-HCV-infected subject's monocytes was remarkably diminished, in comparison with the case where INF-α was not added. Namely, it was verified that the amount of HCV antigens in monocytes was reduced by the action of INF-α. These results suggest that a substance that acts to reduce the amount of HCV antigens in monocytes can be an HCV therapeutic drug in a manner similar to INF-α.

### Example 5 Evaluation of an Anti-Virus Drug Using a Cultured Cell Line

### (1) Establishment of an HCV Infection Using a Cultured Monocyte Cell Line

The premonocyte cell line U937 (ATCC CRL-1593.2: American Type Culture Collection, Manassas, VA, USA) was adjusted to give 1 x 10⁶ cells/mL using RPMI medium supplemented with 10% fetal calf serum, and incubated in a 24-well plate. To the cultured cells were added sera collected from HCV-infected patients (HCV quantitative value: 225 KIU/mL) or sera collected from non-HCV-infected subjects, and incubated at 37°C in the presence of 5% carbon dioxide gas for 2 to 7 days. Subsequently, each cultured U937 cell line was harvested, and immunostaining was performed in the same manner as in Example 1, using the core antibody.

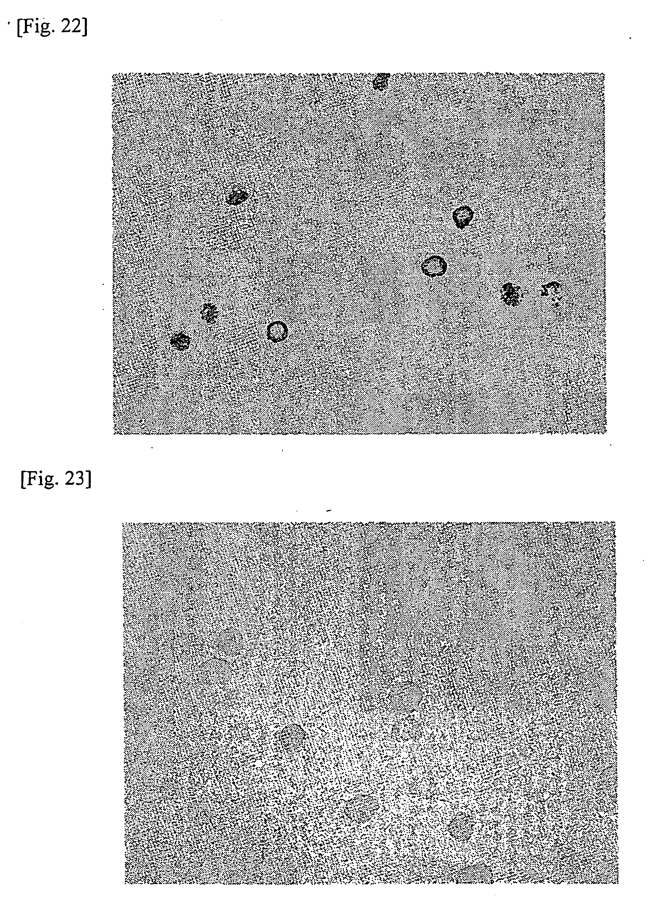

Figure 22 shows the immunostaining result of the U937 cell lines cultured with sera from HCV-infected patients added, and Fig. 23 shows the immunostaining result of the U937 cell lines cultured with sera from non-HCV-infected subjects added. As the figures show, the U937 cell lines that were mixed with the sera from HCV-infected patients had positive immunostaining images (Fig. 22); however, the U937 cell lines that were mixed with the sera from non-HCV-infected subjects did not have positive immunostaining images (Fig. 23).

The results verified that an HCV infection can be established using cultured monocyte cell lines, in the same manner as using peripheral mononuclear cells collected from a living body.

### (2) Evaluation of an Anti-Virus Drug Using Cultured Monocyte Cell Lines

As in (1) above, sera collected from HCV-infected patients (HCV quantitative value: 225 KIU/mL) were added to premonocyte cell lines (U937 cell line) which were cultured in RPMI medium supplemented with 10% fetal calf serum, human INF-α (1 x 10⁵ µg/mL), known anti-HCV therapeutic drug, was further added thereto, and the suspensions were incubated at 37°C in the presence of 5% carbon dioxide gas for 2 to 7 days. As a comparative experiment, U937 cell lines without adding human IFN-α was similarly incubated in RPMI medium supplemented with 10% fetal calf serum at 37°C in the presence of 5% carbon dioxide gas for 2 to 7 days. Each cultured U937 cell line was then harvested, and immunostaining was performed in the same manner as in Example 1, using the core antibody.

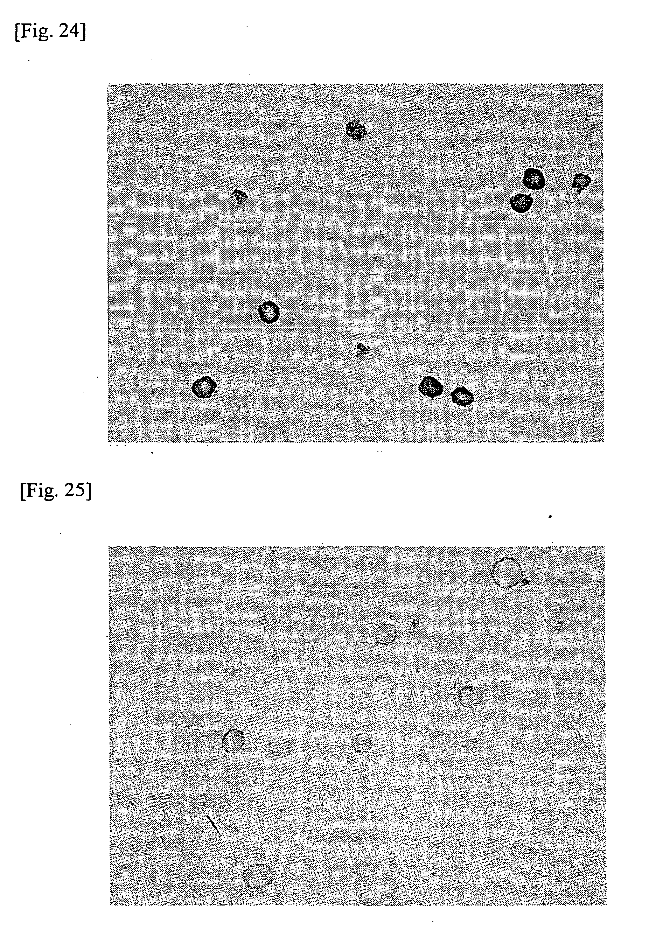

Figure 24 shows the immunostaining result of the U937 cell lines that were cultured without adding IFN-α, and Fig. 25 shows the immunostaining result of the U937 cell lines that were cultured with IFN-α added thereto.

As the results show in Figs. 24 and 25, when INF-α was added to the HCV-infected cultured monocyte cell lines, the immunostain intensity of the cultured monocyte cell lines remarkably decreased, in comparison with the case where INF-α was not added. Namely, it is verified that the amount of HCV antigens in monocytes was reduced by the action of INF-α. This finding suggests that a substance for an HCV therapeutic drug (an anti-HCV drug candidate) can be selected by using cultured monocyte cell lines and by investigating substances that act to reduce the amount of HCV antigens in monocytes.

### Reference Example 6 HCV Removal by Leukocyte-Adsorbing Beads

A 5-ml syringe was filled with 2 g of G-1 beads (JIMRO Co., Ltd., Takasaki, Japan) that aseptically dried beforehand. 1.0 ml of an HCV-infected patient's blood containing heparin was added thereto, and the syringe was set in an RT-5 rotor (TAITEC, 1 rpm) placed in an incubator (37°C) to react for an hour.

After completion of the reaction, the blood inside the syringe was pushed out using a plunger, and the leukocyte counts before and after G-1 bead treatment were measured to determine the number of leukocytes that had been adsorbed onto G-1 beads. Further, the concentrations of HCV nucleic acid and HCV core proteins in the blood before and after the G-1 bead treatment were measured. The HCV nucleic acid concentration was measured by real-time PCR (Roche Diagnostics), and the HCV core protein concentration was measured by an Ortho HCV antigen IRMA test.

The G-1 beads that had adsorbed leukocytes were then placed in a 24-well culture plate, and 1 mL of a 10% FCS-RPMI solution (Gibco, Grand Island NY, USA) was added thereto, followed by incubation in a 5% CO₂ incubator at 37°C for 72 hours. After incubation, the supernatants were collected and the HCV core protein concentrations were measured.

The following results were obtained:
1. The leukocyte count in the blood before G-1 bead treatment was 3,780 cells/µl (n=2) on average, and the leukocyte count in the blood after G-1 bead treatment was 2,070 cells/µl (55%) on average. 45% of the leukocytes were adsorbed onto the G-1 beads.
2. The concentration of HCV nucleic acid in the blood before G-1 bead treatment was 1,680 ± 870 KIU/ml (n=5, average ± standard error), and the concentration thereof after G1 bead treatment was 1,150 ± 610 KIU/ml, thereby showing a reduction to 68% of that before treatment.
3. The concentration of HCV core proteins in the blood before G-1 bead treatment was 6,890 ± 3,530 fmol/L (n=5) on average, and the concentration thereof after G-1 bead treatment was 307 ± 178 fmol/L on average, thereby showing a considerable reduction to 4.5% of that before the treatment.
4. As a result of incubating the leukocyte-adsorbed G-1 beads for 72 hours, HCV core proteins (HCV core antigens) of 17 fmol/L (n=2) on average were detected in the culture supernatant.

The above results revealed that HCV-infected leukocytes can be removed by G-1 bead treatment. Moreover, since HCV was detected in the leukocytes adsorbed onto G-1 beads, it was verified that an HCV treatment which targets leukocytes is effective.

### BRIEF DESCRIPTION OF THE DRAWINGS

[Fig. 1] A microscopic image (200x) showing hepatic tissues that reacted to a monoclonal antibody (NS4 antibody) against HCV NS4 protein, obtained by the experiment described in Example 1 (1).
[Fig. 2] A microscopic image (200x) showing cardiac tissues that reacted to an NS4 antibody, obtained in Example 1 (1).
[Fig. 3] A microscopic image (600x) showing hepatic tissues that reacted to a monoclonal antibody (NS4 antibody) against HCV core protein, obtained in Example 1 (1).
[Fig. 4] A microscopic image (600x) showing cardiac tissues that reacted to a core antibody, obtained in Example 1 (1).
[Fig. 5] A microscopic image (600x) showing renal tissues that reacted to a core antibody, obtained in Example 1 (1).
[Fig. 6] A microscopic image (600x) showing aortal tissues that reacted to a core antibody, obtained in Example 1 (1).
[Fig. 7] A microscopic image (600x) showing lymph node tissues that reacted to a core antibody, obtained in Example 1 (1).
[Fig. 8] A microscopic image (600x) showing bone marrow tissues that reacted to a core antibody, obtained in Example 1 (1).
[Fig. 9] A microscopic image (600x) showing hepatic tissues that reacted to a core antibody in the absence of a core protein, obtained in Example 1 (2).
[Fig. 10] A microscopic image (600x) showing hepatic tissues that reacted to a core antibody in the presence of a core protein, obtained in Example 1 (2).
[Fig. 11] A microscopic image (600x) showing a reaction between monocytes from HCV-infected patients and an NS4 antibody, obtained in Example 2 (1).
[Fig. 12] A microscopic image (600x) showing a reaction between monocytes from non-HCV-infected healthy subjects and NS4 antibodies, obtained in Example 2 (1).
[Fig. 13] A microscopic image (600x) showing a reaction between monocytes from HCV-infected patients and a core antibody, obtained in Example 2 (1).
[Fig. 14] A microscopic image (600x) showing a reaction between monocytes from non-HCV-infected healthy subjects and a core antibody, obtained in Example 2 (1).
[Fig. 15] A microscopic image (600x) showing a non-HCV-infected healthy subject's monocytes which were reacted to serum of an HCV-infected patient, obtained in Example 2 (2).
[Fig. 16] A microscopic image (600x) showing a non-HCV-infected healthy subject's monocytes which were reacted to serum of a non-HCV-infected healthy subject, in Example 2 (2).
[Fig. 17] A microscopic image (600x) showing the immunostaining result of a non-HCV-infected healthy subject's monocytes which were reacted to serum of an HCV-infected patient in the presence of a core antibody, obtained in Example 3.
[Fig. 18] A microscopic image (600x) showing the immunostaining result of a non-HCV-infected healthy subject's monocytes which were reacted to serum of an HCV-infected patient in the presence of an NS4 antibody, obtained in Example 3.
[Fig. 19] A microscopic image (600x) showing the immunostaining result of a non-HCV-infected healthy subject's monocytes which were reacted to serum of an HCV-infected patient in the presence of mouse IgG, obtained in Example 3.
[Fig. 20] A microscopic image (600x) showing the immunostaining result of an HCV-infected patient's monocytes cultured in the absence of interferon-α (IFN-α), obtained in Example 4.
[Fig. 21] A microscopic image (600x) showing the immunostaining result of an HCV-infected patient's monocytes cultured in the presence of IFN-α, obtained in Example 4.
[Fig. 22] A microscopic image (600x) showing the immunostaining result of cultured monocytic cells (U937 cell line) that were cultured with serum of an HCV-infected patient, obtained in Example 5 (1).
[Fig. 23] A microscopic image (600x) showing the immunostaining result of cultured monocytic cells (U937 cell line) that were cultured with serum of a non-HCV-infected patient, obtained in Example 5 (1).
[Fig. 24] A microscopic image (600x) showing the immunostaining result of cultured monocytic cells (U937 cell line) that were reacted to serum of an HCV-infected patient, obtained in Example 5 (2).
[Fig. 25] A microscopic image (600x) showing the immunostaining result of cultured monocytic cells (U937 cell line) that were reacted to serum of an HCV-infected patient in the presence of IFN-α, obtained in Example 5 (2).

## Claims

1. A method for screening anti-hepatitis C virus drug candidates, comprising the following steps (A) to (D):
(A) a step of causing hepatitis C virus to come into contact with monocytes not infected with hepatitis C virus in the presence of a test substance,
(B) a step of measuring the concentration of hepatitis C virus proteins in the monocytes obtained in step (A) (HCV protein concentration),
(C) a step of comparing the HCV protein concentration measured in step (B) with the HCV protein concentration in control monocytes (control HCV protein concentration), which are caused to come into contact with hepatitis C virus in the absence of the test substance, and
(D) a step of selecting the test substance as an anti-hepatitis C virus drug candidate when the HCV protein concentration measured in step (B) is lower than the control HCV protein concentration.

2. A method for screening anti-hepatitis C virus drug candidates, comprising the following steps (a) to (c):
(a) a step of causing a test substance to come into contact with monocytes infected with hepatitis C virus,
(b) a step of measuring the concentration of hepatitis C virus proteins in the monocytes obtained in step (a), and
(c) a step of selecting the test substance as an anti-hepatitis C virus drug candidate when the HCV protein concentration measured in step (b) is lower than the HCV protein concentration in the monocytes before coming into the contact with the test substance, or lower than the HCV protein concentration in control monocytes infected with hepatitis C virus which are not caused to come into contact with the test substance.

3. The screening method according to Claim 1 or 2, wherein an hepatitis C virus protein concentration in monocytes is measured by an immunoassay using an antibody to the hepatitis C virus protein.

## Patentansprüche

1. Verfahren zum Screenen von Anti-Hepatitis-C-Virus-Arzneistoffkandidaten, umfassend die folgenden Schritte (A) bis (D):
(A) Einen Schritt des Verursachens des Inkontaktkommens von Hepatitis-C-Virus mit Monozyten, die nicht mit Hepatitis-C-Virus infiziert sind, in Anwesenheit einer Testsubstanz,
(B) einen Schritt des Messens der Konzentration von Hepatitis-C-Virus-Proteinen in den in Schritt (A) erhaltenen Monozyten (HCV-Proteinkonzentration),
(C) einen Schritt des Vergleichens der HCV-Proteinkonzentration, die in Schritt (B) gemessen wurde, mit der HCV-Proteinkonzentration in Kontroll-Monozyten (Kontroll-HCV-Proteinkonzentration), deren Inkontaktkommen mit Hepatitis-C-Virus in Abwesenheit der Testsubstanz verursacht wurde, und
(D) einen Schritt des Auswählens der Testsubstanz als einen Anti-Hepatitis-C-Virus-Arzneistoffkandidaten, wenn die HCV-Proteinkonzentration, die in Schritt (B) gemessen wurde, niedriger ist als die Kontroll-HCV-Proteinkonzentration.

2. Verfahren zum Screenen von Anti-Hepatitis-C-Virus-Arzneistoffkandidaten, umfassend die folgenden Schritte (a) bis (c):
(a) einen Schritt des Verursachens des Inkontaktkommens einer Testsubstanz mit Monozyten, die mit Hepatitis-C-Virus infiziert wurden,
(b) einen Schritt des Messens der Konzentration von Hepatitis-C-Virus-Proteinen in den in Schritt (a) erhaltenen Monozyten, und
(c) einen Schritt des Auswählens der Testsubstanz als einen Anti-Hepatitis-C-Virus-Arzneistoffkandidaten, wenn die HCV-Proteinkonzentration, die in Schritt (b) gemessen wurde, niedriger ist als die HCV-Proteinkonzentration in den Monozyten bevor sie mit der Testsubstanz in Kontakt gekommen sind, oder niedriger ist als die HCV-Proteinkonzentration in Kontroll-Monozyten, die mit Hepatitis-C-Virus infiziert wurden und deren Inkontaktkommen mit der Testsubstanz nicht verursacht wurde.

3. Screening-Verfahren nach Anspruch 1 oder 2, wobei eine Hepatitis-C-Virus-Proteinkonzentration in Monozyten durch einen Immunoassay unter Verwendung eines Antikörpers gegen das Hepatitis-C-Virus-Protein gemessen wird.

## Revendications

1. Procédé de criblage de médicaments candidats anti-virus de l'hépatite C, comprenant les étapes (A) à (D) suivantes :
(A) une étape consistant à provoquer la mise en contact du virus de l'hépatite C avec des monocytes non infectés par le virus de l'hépatite C en présence d'une substance à tester,
(B) une étape consistant à mesurer la concentration des protéines du virus dé l'hépatite C dans les monocytes obtenus dans l'étape (A) (concentration des protéines du VHC),
(C) une étape consistant à comparer la concentration des protéines du VHC mesurée dans l'étape (B) avec la concentration des protéines du VHC dans les monocytes de contrôle (concentration de contrôle des protéines du VHC), qui sont provoqués pour entrer en contact avec le virus de l'hépatite C en l'absence de la substance à tester, et
(D) une étape consistant à sélectionner la substance à tester en tant que médicament candidat anti-virus de l'hépatite C lorsque la concentration des protéines du VHC mesurée dans l'étape (B) est inférieure à la concentration de contrôle des protéines du VHC.

2. Procédé de criblage de médicaments candidats anti-virus de l'hépatite C, comprenant les étapes (a) à (c) suivantes :
(a) une étape consistant à provoquer la mise en contact d'une substance à tester avec des monocytes infectés par le virus de l'hépatite C,
(b) une étape consistant à mesurer la concentration des protéines du virus de l'hépatite C dans les monocytes obtenus dans l'étape (a), et
(c) une étape consistant à sélectionner la substance à tester en tant que médicament candidat anti-virus de l'hépatite C lorsque la concentration des protéines du VHC mesurée dans l'étape (b) est inférieure à la concentration des protéines du VHC dans les monocytes avant d'entrer en contact avec la substance à tester, ou inférieure à la concentration des protéines du VHC dans les monocytes de contrôle infectés par le virus de l'hépatite C qui ne sont pas provoqués pour entrer en contact avec la substance à tester.

3. Procédé de criblage selon la revendication 1 ou 2, dans lequel la concentration des protéines du virus de l'hépatite C dans les monocytes est mesurée par un dosage immunologique utilisant un anticorps dirigé contre les protéines du virus de l'hépatite C.
